# EUROPEAN PATENT SPECIFICATION

(11) **EP 3 803 358 B1**
(45) Date of publication and mention of the grant of the patent: **06.09.2023**
(21) Application number: 19737256.8
(22) Date of filing: 04.06.2019
(51) Int. Cl.: G01N 27/02, G01N 33/42

(54) **METHOD FOR PREDICTING BITUMEN PROPERTIES**
VERFAHREN ZUR VORHERSAGE VON BITUMENEIGENSCHAFTEN
PROCÉDÉ DE PRÉDICTION DE PROPRIÉTÉS DE BITUME

(30) Priority: 04.06.2018 IN 201841020816
(43) Date of publication of application: 14.04.2021
(73) Proprietor: Shell Internationale Research Maatschappij B.V., 2596 HR The Hague (NL)
(72) Inventor: D'MELO, Dawid John, Bangalore North, Karnataka, 562149 (IN); RAO, Girish, Bangalore North, Karnataka, 562149 (IN); TAYLOR, Richard Ernest, London SE1 7NA (GB); PARK, Timothy Alexander, 1031HW Amsterdam (NL); BHATTACHARYA, Subhendu Manindra, Bangalore North, Karnataka, 562149 (IN)
(74) Representative: Shell Legal Services IP
(86) International application number: PCT/IN2019/050433
(87) International publication number: WO 2019/234762

(56) References cited:
- W. H. HUNTER WOODWARD ET AL: "Method for Estimating Oil Viscosity via Dielectric Spectroscopy", ENERGY & FUELS., vol. 28, no. 9, 19 August 2014 (2014-08-19), pages 5707-5713, XP055619864, WASHINGTON, DC, US. ISSN: 0887-0624, DOI: 10.1021/ef501362r
- ERIC Y. SHEU ET AL: "Dielectric Response of Asphaltenes in Solvent", ENERGY & FUELS., vol. 8, no. 3, 1 May 1994 (1994-05-01), pages 552-556, XP055620323, WASHINGTON, DC, US. ISSN: 0887-0624, DOI: 10.1021/ef00045a005
- FENG CHEN ET AL: "A study on dielectric response of bitumen in the low-frequency range", ROAD MATERIALS AND PAVEMENT DESIGN, vol. 16, no. sup1, 25 May 2015 (2015-05-25), pages 153-169, XP055619867, ISSN: 1468-0629, DOI: 10.1080/14680629.2015.1029682

## Description

### Field of the Invention

The invention relates to a method for predicting bitumen properties using dielectric spectroscopy.

### Background of the Invention

Bitumen consists essentially of hydrocarbons and their derivatives. Depending on its source, at ambient temperatures bitumen can be a viscous liquid or a solid. Its physical state softens gradually when heated, making it a useful material, especially for construction applications. For example, bitumen is used as a binder in a variety of applications ranging from roofing, flooring to sealing. Bitumen may be combined with aggregates to provide asphalt that can be used for, for example, manufacturing roads and airport runways.

Bitumen is a substance with diverse qualities, and such qualities must be suitable for any one of the diverse applications that it can be used for. For example, for road paving application, the bitumen used to make asphalt must conform to strict specification systems that are set by authorities in each country or region around the world, in line with the physical stresses a given road will be exposed to during its lifetime (such as the extent of loads it must bear to the range of temperatures that the road will be exposed to etc.).

To assess the suitability of bitumen and to characterise it, for example, for road paving applications, analytical methods exist for assessing its various properties, such as, but not limited to change in softening point, softening point, penetration, upper performance grading ('PG grading') temperature, p-value, asphaltene content, m-value, change in softening point after Rolling Thin-Film Oven Test ('RTFOT') ageing, wax content, kinematic and dynamic viscosities, Fraass breaking point and viscosity based ageing index.

The specification systems used across the world in bitumen product standards can be broadly described as either empirical or performance based.

The specification systems based on empirical testing, such as penetration, softening point and viscosity, have been used for many decades. Penetration and the ring and ball softening point test are used as the main characteristics in product standards throughout Europe, many parts of Asia (including China), Africa and Argentina.

The performance based specification system (Performance Grading, or 'PG grading') used in the USA is well established, and individual regulators and specifiers can determine the grades to be used on state roads. In countries such as China, PG grading is used for premium binders and for construction projects such as airfields, motor sport circuits, heavy duty expressways or highways exposed to extreme weather conditions. For conventional bitumen specifications, China uniquely employs ductility (at both normal temperature and low temperature).

Viscosity based ('VG') standards are used more commonly in Australia and New Zealand, and for the softer grades in Europe, India and South America.

Derivations, combinations and local adaptations of these standards are used depending on the regulatory environment, climate and historical factors. Even when regional product standards are used (e.g. the harmonised norms in Europe), differences still exist between countries. Further, other performance based systems are under development in many countries.

Therefore, the manufacturers, suppliers and end-users across the bitumen supply chain must be able to assess the suitability of any particular bitumen for any given application.

However, the known analytical methods performed on bitumen require specialised laboratory equipment and trained technicians to undertake them. Further, the undertaking of numerous analytical methods to assess multiple properties of bitumen is costly and time consuming.

In view of this, attempts have been made to reduce the burden of undertaking laboratory tests by seeking to predict bitumen properties in a more efficient way, for example, by the use of bitumen's intrinsic properties due it is molecular composition.

Walther, H. in "Zur Analyse dielektrischer Dispersionkuvern bituminöser Stoffe" Kollid Z. (1950) v. 117, p. 75, reported that measuring permittivities of bitumens over a wide temperature range gave results which were of value in interpreting rheological properties, because structure and permittivity are related.

Heithaus, J.J. in "Measurement and significance of asphaltene peptization" J. Inst. Petrol. (1962) vol. 48, p. 45, reported that permittivity values of dilute solutions of maltenes and asphaltene varied in the same manner as 'flocculation ratio', an indication of bitumen durability.

UK's Transport and Road Research Laboratory (TRRL) Laboratory Report 777 (1977) (ISBN 0305-1293) reported a good correlation between flocculation ratio and with petroleum bitumen's ability to produce texture in asphalt, and in view of Walther (1950) and Heithaus (1962), the authors measured the permittivities of solid bitumen samples extracted from asphalt core samples, and found that their compatibility with oils, and their resistance to weathering, both related to their permittivity. The permittivities of bitumen samples extracted from asphalt core samples were measured at 1590 Hz, and the authors discouraged the use of higher frequencies by teaching that at high frequencies, the more massive molecular aggregations cannot move rapidly enough to follow the electric field. Further, the authors taught that the permittivity test must be restricted to petroleum bitumen containing no additives, and that only bitumen alone can be cast (solidified) in a parallel-plate borosilicate-glass cell.

WO1997014953 concerns the use of statistical analysis, or a neural network, to generate a model to predict a physical property of a residual hydrocarbonaceous material by correlating its near infrared spectra with its physical properties measured by conventional means.

GB1381921 concerns a portable non-destructive testing apparatus for determining the density of an in-place material by moving the apparatus across the surface of the material and measuring variations in the dielectric strength of the material.

W.H. Hunter Woodward, et al., "Method for Estimating Oil viscosity via Dielectric Spectroscopy", Energy & Fuels, Vol 28, No. 9, 19 August 2014, 5707-5713 discloses a method for estimating the viscosity of extra-heavy oil that occurs naturally as Canadian 'Oil Sands', in order to prepare it for its transport via pipeline to refineries.

Eric Y. Sheu et al., "Dielectric Response of Asphaltenes in Solvent", Energy and Fuels, Vol 8, No. 3, 1 May 1994, pages 552-556 describes a dielectric relaxation study of asphaltenes in toluene, to elucidate the mechanism and structure of how they aggregate into micellular structures and the nature of their electrical dipoles.

Feng Chen et al., "A study on dielectric response of bitumen in the low-frequency range", Road Materials and Pavement Design, vol. 16, No. supl., 25 May 2015, pages 153-169 compares the temperature and frequency dependency of the dielectric constants of solid bitumen (alone) and solid wax-modified bitumen.

An object of the present invention is to predict bitumen properties in a more efficient way, by avoiding numerous lengthy and costly laboratory analytical methods. By performing a single test, the present invention enables the prediction the levels of multiple properties of bitumen. The method of the invention is quick, simple and avoids the need for multiple different tests to be performed on bitumen to assess its different properties.

### Summary of the Invention

Accordingly, the present invention provides a method for predicting one or more properties of bitumen using dielectric spectroscopy, wherein the method comprises: (a) dissolving a bitumen sample in a solvent to form a bitumen solution; (b) measuring the impedance response of the bitumen solution across a frequency range of from 1,400 Hz to 1,550 Hz and additionally across a frequency range of from 2,000 Hz to 3,000 Hz and/or across a frequency range of from 51,000 Hz to 54,000 Hz; (c) converting the impedance response of the bitumen solution to a dielectric loss factor; and (d) comparing the dielectric loss factor of the bitumen solution to a reference database comprising dielectric loss factors of reference bitumen samples to predict one or more properties of the bitumen sample comprising the bitumen solution.

The present invention enables the prediction of multiple properties of a bitumen sample such as, but not limited to, its change in softening point, softening point, penetration, upper performance grade ('PG') temperature (untreated, and after Rolling Thin-Film Oven Test ('RTFOT')), p-value, asphaltene content, m-value, change in softening point after Thin-Film Oven Test (`TFOT') ageing, wax content, kinematic and dynamic viscosities, Fraass breaking point and/or viscosity based ageing index, using only a single value, namely its dielectric loss factor/value.

### Brief Description of the Drawings

Figure 1 shows the correlation between the dielectric loss factor and the softening point of bitumen.
Figure 2 shows the correlation between the dielectric loss factor and the change in softening point of bitumen.

### Detailed Description of the Invention

Due to the different electrical behaviours of different materials , which are in turn based on their molecular composition, measuring electrical properties of materials can provide a way of differentiating between them.

One way of differentiating materials by their electrical properties is by the use of 'dielectric spectroscopy', a technique that measures the impedance of materials as a function of frequency.

Dielectric spectroscopy is used in the method according to the present invention. The impedance responses/values of bitumen samples in solution, at a frequency, or varying across a range of frequencies, can be measured using an impedance analyser/dielectric spectrometer. Any commercially available impedance analyser may be used in the method according to the present invention, such as a 'Sciospec ISX-3 mini'.

The impedance analyser uses a parallel combination of resistor and capacitor to assess the material under investigation (e.g. the bitumen sample in solution). Once the circuit components are known, for example using a Cole-Cole plot, then both the dielectric loss factor, and permittivity, of the bitumen sample in solution can be calculated from the dielectric spectrometer's data output.

In the method of the present invention, a bitumen sample of interest is dissolved in a solvent to form a bitumen solution.

The solvent should not interfere with the measurement of the impedance value/response of the bitumen samples, and thus the dielectric constant of the solvent should not change significantly with changes in the frequency of measurement of the impedance response.

Suitably, the solvent may be an aromatic solvent, a chlorinated solvent or a heterocyclic organic solvent. More particularly, the aromatic solvent may be selected from, but not limited to, toluene, xylene, α-methyl naphthalene or cyclohexane. Alternatively, the chlorinated solvent may be selected from, but not limited to, dichloromethane, tetrachloroethylene, trichloroethylene. Alternatively, the heterocyclic organic solvent may be selected from, but not limited to, tetrahydrofuran. Preferably the solvent is xylene, toluene or tetrahydrofuran. More preferably the solvent is xylene.

The bitumen sample of interest may be dissolved in a solvent, such that the resultant bitumen solution comprises from 1%wt. to 40 %wt. bitumen. Preferably, the bitumen solution comprises at least 2 %wt. bitumen, more preferably at least 3 %wt. bitumen, even more preferably at least 4 %wt. bitumen, most preferably at least 4.5 %wt. bitumen. Preferably, the solution of bitumen comprises at most 20 %wt. bitumen, more preferably at most 10 %wt. bitumen, even more preferably at most 8 %wt. bitumen, even more preferably at most 6 %wt., most preferably at most 5.5 %wt. bitumen.

Even most preferably, the solution of bitumen comprises 5 %wt. bitumen.

The impedance measurements may be obtained at a sample temperature of about 20-60 °C, preferably 22-25 °C.

Dielectric spectroscopy is used in the method according to the present invention. Dielectric spectroscopy generally measures the impedance and the phase angle of a tested material (together the 'impedance response'), at a particular frequency, or across varying frequencies. Impedance is the opposition to flow of alternating current in a complex system. Phase angle is a measure of the delay between the application of a voltage across a sample compared to the current induced through it.

The impedance responses of bitumen samples in a solvent (i.e. 'bitumen solution' prepared according to the present invention) were measured across a frequency range of from 100 Hz to 1 MHz. The measured impedance values were then converted to its 'real' and its 'imaginary' parts using Circuit Theory Analogy.

Using 'Cole-Cole' plot analysis, the 'imaginary' part of the impedance responses so obtained was plotted against the 'real' part of impedance, or alternatively, the 'imaginary' part of impedance was plotted against frequency, in both cases to evaluate and to elucidate the samples' relaxation peak(s), and to assess their resistive and capacitive elements. Cole-Cole plot analysis would be readily known to the skilled person and can also be found in, for example, Cole, K.S. and Cole, R.H. (1941). "Dispersion and Absorption in Dielectrics - I Alternating Current Characteristics" J. Chem. Phys. 9 (4): 341-352, and/or, Cole, K.S. and Cole, R.H. (1942) "Dispersion and Absorption in Dielectrics - II Direct Current Characteristics" Journal of Chemical Physics. 10 (2): 98-105. In the case of bitumen solutions prepared as described herein, a single relaxation peak was observed for each sample, indicating that each behaved as a 'single circuit element'. Alternatively, commercial software, such as 'WinFIT'^{™} curve fitting software for non-linear models, or 'ZSimpWin' electrochemical Impedance Spectroscopy (EIS) Data Analysis software can be used for converting impedance measurements to capacitance and resistance, as well as to undertake, for example, a Cole-Cole plot analysis.

Using Circuit Theory Analogy with the assumption that such bitumen solutions behave as a single circuit element, the capacitance and resistance values thus obtained were then converted to dielectric loss factor and permittivity values across said frequency range. The use of Circuit Theory Analogy and undertaking such calculations would be readily known to the skilled person and can also be found in V.F. Lvovich, M.F. Smiechowski, Electrochimica Acta 51 (2006) 1487-1496 and Impedance Spectroscopy: Applications to Electrical and Dielectric Phenomena, Vadim F. Lvovich, Pub: John Wiley & Sons, Inc. (2012).

Principal Component Analysis ('PCA') was then used to determine the preferred frequency or frequencies for obtaining impedance values that can be used to distinguish between bitumen properties of interest, and their levels/values (e.g. 'softening point' of the bitumen and its value). PCA would be readily known to the skilled person and can also be found in, for example, Pearson, K. (1901) "On Lines and Planes of Closest Fit to Systems of Points in Space" Philosophical Magazine. 2 (11): 559-572, Hotelling, H. (1933) "Analysis of a complex of statistical variables into principal components" Journal of Educational Psychology, 24, 417-441, and 498-520, and Hotelling, H (1936) "Relations between two sets of variates" Biometrika 28 (3/4): 321-377.

The dielectric loss factors derived from such preferred frequency or frequencies was/were then used to develop regression models to correlate dielectric loss factor values so obtained to the levels/values of different bitumen properties that were also measured using conventional laboratory techniques. Such a regression model can be developed using, for example, the 'Least Absolute Shrinkage and Selection Operator' ('LASSO'), or any other linear regression technique known in the art. The LASSO model that was used not only indicated that the regression in the present case was linear, but also provided a measure of the accuracy with which different bitumen properties could be predicted from different frequency/frequencies.

Data from the regression analysis was captured in a database, such that an input of the dielectric loss values obtained from any bitumen solution according to the method herein can be converted to the level/value of a bitumen property of interest using such database as a reference database.

To generate the reference database and to enable this comparison, the inventors of the present method carried out conventional analysis of multiple reference bitumen samples with different change in softening points, different softening points, different penetrations, different upper performance grade ('PG') temperature (untreated, and after Rolling Thin-Film Oven Test ('RTFOT', different p-values, different asphaltene contents, different m-values, different change in softening points after TFOT ageing, different wax contents, different kinematic and dynamic viscosities, different Fraass breaking points and different viscosity based ageing index, to work out the multiple different properties of each reference bitumen sample. The dielectric loss factors of each of these reference bitumen samples were also calculated using the process described above, and inputted to the database.

Although the response of a material to an applied electric field is the highest at the relaxation peak, the single relaxation peak observed in the case of bitumen samples prepared according to the present invention was found not to correspond to a frequency or frequencies that provided the highest level/accuracy of bitumen property prediction. Instead, by their analysis across the frequency range of from 100 Hz to 1MHz, when predicting bitumen properties using dielectric loss factor, the inventors surprisingly discovered that the impedance response of the bitumen samples prepared according to the invention provided the highest level of accuracy when measured across a frequency range of from 1 1,400 Hz to 1,550 Hz, or more preferably from 1,480 Hz to 1,520 Hz. Alternatively, the frequency of 1,500 Hz may be used.

More surprisingly, inventors discovered that the accuracy of the prediction derived from said frequency ranges, or frequency, can be improved by about 3-4%, if additionally, impedance measurement(s) so obtained are/is combined with impedance response data of the bitumen samples measured across a frequency range of from 2,000 Hz to 3,000 Hz, or preferably from 2,350 Hz to 2,450 Hz, or alternatively, data from the frequency of 2,416 Hz may be used.

A further improvement of about 3-4% was also discovered when impedance responses from any of the aforementioned impedance response data are/is combined with impedance response data across a frequency range of from 51,000 Hz to 54,000 Hz, or alternatively, combined with data from the frequencies of 51,392 Hz and/or 53,521 Hz. Measurement of impedance values across a frequency range of from 1,590 to 1,990 Hz did not provide significant improvement of bitumen property prediction.

In an exemplary method impedance response of the bitumen solutions may be obtained from the frequency range of from 1,400 Hz to 1,550 Hz, or more preferably from 1,480 Hz to 1,520 Hz, or from the frequency of 1,500 Hz and combined with data obtained from the 2,000 Hz to 3,000 Hz range, or its abovementioned narrower ranges, or from the frequency of 2,416 Hz. Such combined data may be further combined with data from the frequency range of from 51,000 Hz to 54,000 Hz, or alternatively, combined with data from the frequencies of 51,392 Hz and/or 53,521 Hz. It is also possible to combine the data from the frequency range of from 1,400 Hz to 1,550 Hz, or more preferably from 1,480 Hz to 1,520 Hz, or at the frequency of 1,500 Hz with data from a frequency range of from 51,000 Hz to 54,000 Hz, or alternatively, with data from the frequencies of 51,392 Hz and/or 53,521 Hz, without combining it with data from 2,000 Hz to 3,000 Hz range.

The inventors also assessed whether permittivity could be used to predict bitumen properties. Whereas dielectric loss factor of a material is a measure of its inherent dissipation of electromagnetic energy, generally by heat or by delayed re-orientation of dipoles with change in frequency, permittivity is a material's ability to resist an electric field, being a ratio of the capacitance of the material to the capacitance of air. The inventors found that the numerical spread of permittivity data obtained from the bitumen samples prepared as described herein was not wide enough to distinguish between different samples and their properties, and further, did not provide suitable level of accuracy for predicting bitumen properties.

The inventors of the present invention observed that dielectric loss factor provided a good resolution between the impedance values of the bitumen samples in solution, thus enabling an improved and efficient method for predicting multiple properties of bitumen with higher levels of accuracy.

### Examples

The invention will now be described by reference to examples which are not intended to be limiting of the invention.

### Example 1

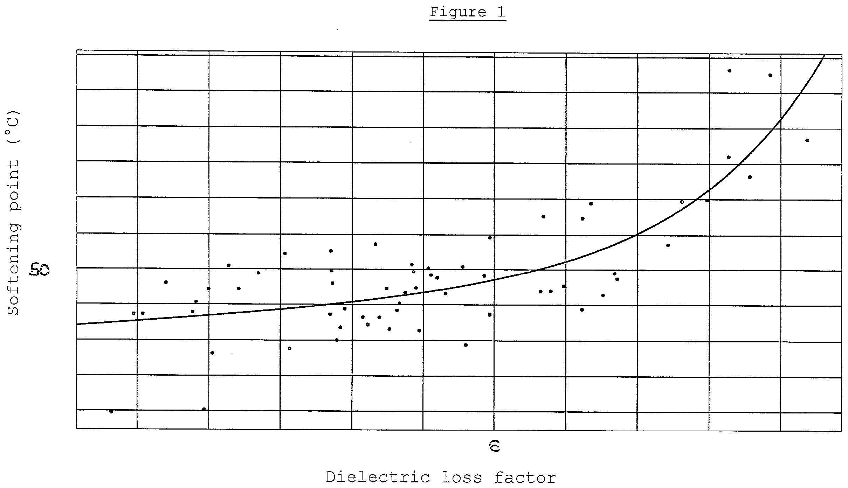

In this example, the correlation between the dielectric loss factors and the softening points of multiple reference bitumen samples was assessed and plotted as shown in Figure 1. A LASSO regression technique was used to fit a two-stage linear regression model.

### Example 2

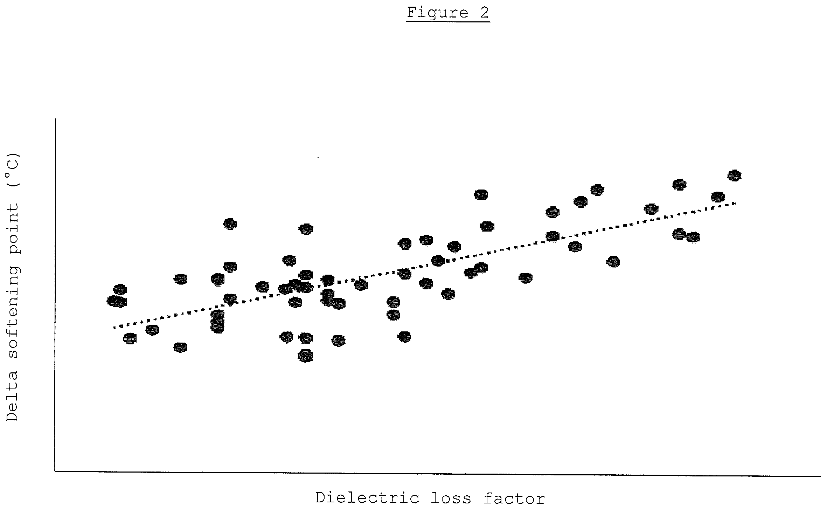

In this example, the correlation between the dielectric loss factors and the change in softening points of multiple reference bitumen samples was assessed and plotted as shown in Figure 2. A LASSO regression technique was used to fit a linear regression model.

### Example 3

In this example, an impedance measurement was performed on a 5 % wt./vol bitumen solution in toluene at a voltage amplitude of 0.5 V to 2.5 V, at a frequency in the range of from 1,480 Hz to 1,520 Hz, and at 1,500 Hz. The impedance responses were converted to dielectric loss factors as describe herein. The dielectric loss factor values were converted to softening point using, for example, the plot shown in Figure 1, such that a dielectric loss value of about 6 at 1,500 Hz, gave a softening point of about 48 °C (+/- 3 °C, at about 75 % accuracy). Similar conversions were carried out using plots of, for example, change in softening point (as per Figure 2). Additional data from the frequency range of from 2,350 Hz to 2,450 Hz provided about 3-4% additional accuracy. Similar level of additional accuracy was also obtained by combining data from the frequency range of from 51,000 to 54,000 Hz, or from the frequencies of 51,392 Hz and/or 53,521 Hz.

Alternatively, the dielectric loss factor of the sample may be compared the reference database compiled as described herein.

## Claims

1. A method for predicting one or more properties of bitumen using dielectric spectroscopy, wherein the method comprises:
(a) dissolving a bitumen sample in a solvent to form a bitumen solution;
(b) measuring the impedance response of the bitumen solution across a frequency range ;
(c) converting the impedance response of the bitumen solution to a dielectric loss factor; and
(d) comparing the dielectric loss factor of the bitumen solution to a reference database comprising dielectric loss factors of reference bitumen samples to predict one or more properties of the bitumen sample comprising the bitumen solution;
**characterised in that**
measuring in step (b) is across a frequency range of from 1,400 Hz to 1,550 Hz and additionally across a frequency range of from 2,000 Hz to 3,000 Hz and/or across a frequency range of from 51,000 Hz to 54,000 Hz.

2. The method according to Claim 1, wherein at step (c), circuit theory analogy is used to convert the measured impedance response into the dielectric loss factor.

3. The method according to any one of Claims 1 or 2, wherein the bitumen solution of step (a) comprises from 2 %wt. to 10 %wt. bitumen.

4. The method according to any one of Claims 1 to 3, wherein the one or more properties of the bitumen to be predicted include change in softening point, softening point, penetration, upper performance grade ('PG') temperature (untreated, and after Rolling Thin-Film Oven Test ('RTFOT')), p-value, asphaltene content, m-value, change in softening point after Thin-Film Oven Test (TFOT) ageing, wax content, kinematic and dynamic viscosities, Fraass breaking point and/or viscosity based ageing index.

5. The method according to Claim 4, wherein step (b) is performed using a dielectric spectrometer.

## Patentansprüche

1. Verfahren zum Vorhersagen einer oder mehrerer Eigenschaften von Bitumen unter Verwendung von dielektrischer Spektroskopie, wobei das Verfahren umfasst:
(a) Auflösen einer Bitumenprobe in einem Lösungsmittel, um eine Bitumenlösung auszubilden;
(b) Messen der Impedanzreaktion der Bitumenlösung über einen Frequenzbereich;
(c) Umwandeln der Impedanzreaktion der Bitumenlösung auf einen dielektrischen Verlustfaktor; und
(d) Vergleichen des dielektrischen Verlustfaktors der Bitumenlösung mit einer Referenzdatenbank, umfassend dielektrische Verlustfaktoren von Referenzbitumenproben, um eine oder mehrere Eigenschaften der Bitumenprobe, umfassend die Bitumenlösung, vorherzusagen;
**dadurch gekennzeichnet, dass**
ein Messen in Schritt (b) über einen Frequenzbereich von 1.400 Hz bis 1.550 Hz und zusätzlich über einen Frequenzbereich von 2.000 Hz bis 3.000 Hz und/oder über einen Frequenzbereich von 51.000 Hz bis 54.000 Hz erfolgt.

2. Verfahren nach Anspruch 1, wobei in Schritt (c) die Schaltungs-Theorie-Analogie verwendet wird, um die gemessene Impedanzreaktion in den dielektrischen Verlustfaktor umzuwandeln.

3. Verfahren nach einem der Ansprüche 1 oder 2, wobei die Bitumenlösung von Schritt (a) zu 2 Gew.-% bis 10 Gew.-% Bitumen umfasst.

4. Verfahren nach einem der Ansprüche 1 bis 3, wobei die eine oder die mehreren Eigenschaften des Bitumens, das vorhergesagt werden soll, eine Änderung des Erweichungspunkts, einen Erweichungspunkt, eine Penetration, eine Temperatur des oberen Leistungsgrads ("PG"-Temperatur) (unbehandelt und nach dem Rollen-Dünnfolienofentest ("RTFOT")), einen p-Wert, einen Asphalten-Gehalt, einen m-Wert, eine Änderung des Erweichungspunktes nach der Dünnfolienofentest-Alterung (TFOT-Alterung), einen Wachsgehalt, kinematische und dynamische Viskositäten, einen Fraass-breaking-point und/oder einen viskositätsbasierten Altersindex einschließen.

5. Verfahren nach Anspruch 4, wobei Schritt (b) unter Verwendung eines dielektrischen Spektrometers durchgeführt wird.

## Revendications

1. Procédé destiné à prédire une ou plusieurs propriétés du bitume à l'aide d'une spectroscopie diélectrique, dans lequel le procédé comprend :
(a) la dissolution d'un échantillon de bitume dans un solvant pour former une solution de bitume ;
(b) la mesure de la réponse d'impédance de la solution de bitume à travers une plage de fréquences ;
(c) la conversion de la réponse d'impédance de la solution de bitume en un facteur de perte diélectrique ; et
(d) la comparaison du facteur de perte diélectrique de la solution de bitume à une base de données de référence comprenant des facteurs de perte diélectrique d'échantillons de bitume de référence pour prédire une ou plusieurs propriétés de l'échantillon de bitume comprenant la solution de bitume ;
**caractérisé en ce que**
la mesure de l'étape (b) est à travers une plage de fréquences allant de 1400 Hz à 1550 Hz et en complément à travers une plage de fréquences allant de 2000 Hz à 3000 Hz et/ou à travers une plage de fréquences allant de 51 000 Hz à 54 000 Hz.

2. Procédé selon la revendication 1, dans lequel à l'étape (c), l'analogie de la théorie des circuits est utilisée pour convertir la réponse d'impédance mesurée en le facteur de perte diélectrique.

3. Procédé selon l'une quelconque des revendications 1 ou 2, dans lequel la solution de bitume de l'étape (a) comprend de 2 % en poids à 10 % en poids de bitume.

4. Procédé selon l'une quelconque des revendications 1 à 3, dans lequel la ou les propriétés du bitume à prédire comportent une variation de point de ramollissement, un point de ramollissement, un pénétration, un classement de performances supérieures (« PG ») (non traité, et après test au four de laminage de couches minces (« RTFOT »)), la valeur p, la teneur en asphaltènes, la valeur m, le changement de point de ramollissement après un vieillissement par test au four de couches minces (TFOT), une teneur en cire, des viscosités cinématique et dynamique, un point de rupture de Fraass et/ou un indice de vieillissement basé sur la viscosité.

5. Procédé selon la revendication 4, dans lequel l'étape (b) est réalisée à l'aide d'un spectromètre diélectrique.
